# EUROPÄISCHE PATENTSCHRIFT

(11) **EP 0 595 891 B1**
(45) Veröffentlichungstag und Bekanntmachung des Hinweises auf die Patenterteilung: **15.03.1995**
(21) Anmeldenummer: 92915538.0
(22) Anmeldetag: 13.07.1992
(51) Int. Cl.: E01C 19/28, E02D 3/02

(54) **VERFAHREN ZUM FESTSTELLEN UND ANZEIGEN DER BEIM ARBEITEN MIT EINEM BODENVERDICHTUNGSGERÄT ERREICHTEN BODENDICHTE**
PROCESS FOR DETERMINING AND INDICATING THE DEGREE OF GROUND COMPACTING ATTAINED DURING WORK WITH A GROUND COMPACTER
PROCEDE DE DETERMINATION ET D'INDICATION DE LA DENSITE DU SOL OBTENUE PAR UN ENGIN DE COMPACTAGE DU SOL

(30) Priorität: 20.07.1991 DE 4124193
(43) Veröffentlichungstag der Anmeldung: 11.05.1994
(60) Teilanmeldung: 94112628.6
(73) Patentinhaber: WACKER-WERKE GMBH & CO. KG, D-80809 München (DE)
(72) Erfinder: STEFFEN, Michael, D-8035 Gauting (DE)
(74) Vertreter: Hieke, Kurt
(86) Internationale Anmeldenummer: EP9201586
(87) Internationale Veröffentlichungsnummer: WO9302253

(56) Entgegenhaltungen:
- EP-A- 0 092 484
- EP-A- 0 281 683
- PATENT ABSTRACTS OF JAPAN volume 15, No 279 (M-1136) July 1991 & JP-A- 30 96 637 (MAZDA MOTOR CORPORATION)

## Beschreibung

Die Erfindung bezieht sich auf ein Verfahren gemäß dem Oberbegriff des Patentanspruchs 1.

Bodenverdichtungsgeräte werden in der Regel mit konstanter Stellung des Liefergradhebels, d.h. des die Energiezufuhr steuernden Hebels, betrieben.

Es ist bekannt (z.B. aus dem Dokument EP-A-0 281 683), zur Echtzeit-Feststellung und -Anzeige des beim Arbeiten mit einem Bodenverdichtungsgerät erreichten Grades der Bodendichte einen die Belastung des Motors wiederspiegelnden Motorparameter zu überwachen und in ein entsprechendes elektrisches Signal umzusetzen. Geeignete Betriebsparameter sind in diesem Zusammenhang bei Verbrennungskraftmaschinen z.B. das von der Motorwelle übertragene Drehmoment und die Ladung des Motors.

Die direkte Erfassung des jeweiligen Betriebsparameters mit guter Genauigkeit insbesondere in dem bei Bodenverdichtungsarbeiten besonders interessanten Endbereich des anzustrebenden Verdichtungsgrades ist jedoch bisher schwierig gewesen und erforderte einen hohen baulichen Aufwand.

Heutzutage werden Verdichtungsgeräte häufig mit Motoren angetrieben, die über eine Überdrehzahl-Schutzeinrichtung verfügen.

Aufgabe der Erfindung ist es, ein Verfahren gemäß dem Oberbegriff des Patentanspruchs 1 zu schaffen, das mit einem verhältnismäßig kleinen baulichen Aufwand durchführbar ist und sehr genaue Ergebnisse insbesondere im Endbereich der anzustrebenden Bodenverdichtung, also dort, wo die Entscheidung zu treffen ist, mit der Verdichtungsarbeit aufzuhören, liefert.

Die vorstehende Aufgabe wird durch die im Patentanspruch 1 genannten Merkmale gelöst.

Der über eine bestimmte Zeitdauer gemittelte Wert der Spätverstellung der Zündimpulse ist kein direkter Betriebsparameter des Motors, spiegelt aber dennoch den augenblicklichen Lastfall des Motors insbesondere zum Ende der Verdichtung eines gerade in Bearbeitung befindlichen Bodens hin sehr genau wieder und läßt sich auch auf besonders einfache Weise in ein entsprechendes elektrisches Signal umsetzen.

Die abhängigen Ansprüche betreffen bevorzugte Ausgestaltungen des Verfahrens nach Anspruch 1.

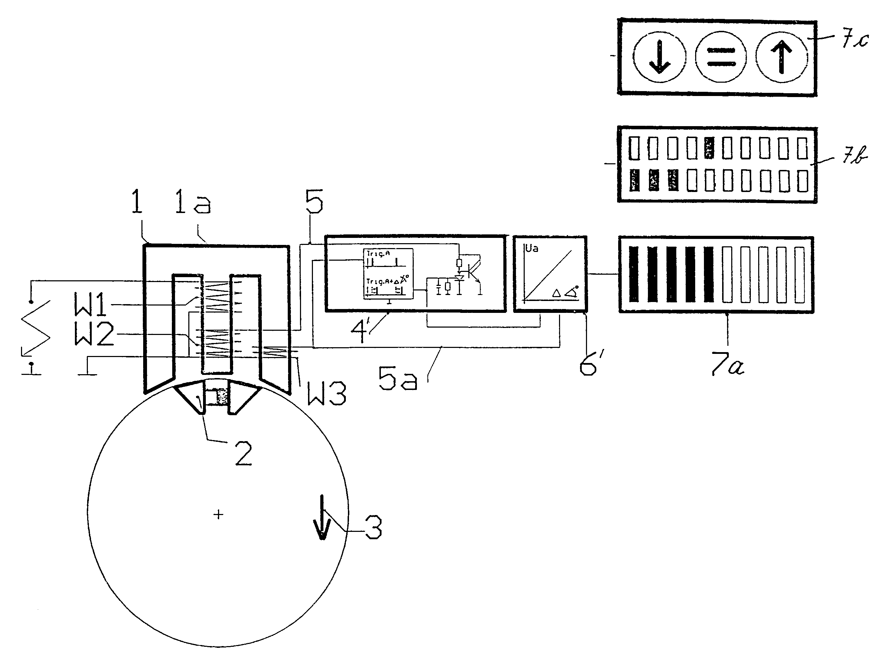

Die Erfindung wird nachstehend anhand der Zeichnung an einem Ausführungsbeispiel, das in der einzigen Figur der Zeichnung schematisch dargestellt ist, noch näher erläutert.

In der Zeichnung sind jeweils nur die Zündelektronik mit ihren vom Motor angetriebenen Teilen sowie die Einrichtung zum Erfassen der Spätverstellung bzw. des Spätverstellwinkels und zu deren Umwandlung in ein entsprechendes Anzeigesignal dargestellt.

Der im einzelnen nicht dargestellte Motor kann als solcher von üblicher Bauart sein.

Bei dem von dem Motor angetriebenen, ebenfalls nicht dargestellten Bodenverdichtungsgerät kann es sich um eine Verdichtungsplatte, einen Stampfer, eine Walze oder dgl. von bekannter Konstruktion handeln.

Bei dem in der Zeichnung dargestellten Ausführungsbeispiel weist die Zündanlage für den Motor einen mit einem E-Kern 1a versehenen Zündtransformator 1 auf, der auf zwei von den drei Kernschenkeln drei Spulen W1, W2 und W3 trägt. Die Schenkel des Magnetkerns 1a werden stirnseitig paarweise periodisch durch ein sich der Reihe nach an den Kernschenkeln vorbeibewegendes "Joch" 2 überbrückt, das drehzahlsynchron mit der Nockenwelle in der durch den Pfeil 3 angedeuteten Richtung umläuft. Die Spulen W1 und W2 sind auf dem mittleren Kernschenkel angeordnet, die Spule W3 auf dem in Umlaufrichtung des Jochs 2 hinten liegenden äußeren.

Die Spule W1 ist die die jeweilige Zündkerze ggf. über einen nicht dargestellten Verteiler beaufschlagende Hochspannungs-Sekundärwicklung, und die Wicklung W₂ ist die zugehörige, von der Zündelektronik 4 beaufschlagte Primärwicklung. Die Wicklung W₃ liefert an die Zünd-Elektronik 4' ein drehzahlproportionales elektrisches Signal. Die Zünd-Elektronik 4' bewirkt eine Drehzahl-Endbegrenzung, so daß der Motor einen vorbestimmten Drehzahlwert nur geringfügig überschreiten kann. Zu diesem Zweck ist die Zündelektronik 4' so gestaltet, daß sie zwar eine der Nockenwellendrehzahl entsprechende Anzahl von Zündimpulsen an die Zündkerzen liefert, den Zündzeitpunkt aber zum vorbestimmten Drehzahlmaximalwert hin progressiv später stellt.

Der von der Wicklung W3 gelieferte Puls und der von der Zündelektronik 4' an die Zündkerzen gelieferte Puls werden einem Rechner 6' zugeführt, und dieser bildet eine Gleichspannung, die der jeweils über ein vorbestimmtes Zeitintervall gemittelten Größe der Phasenverschiebung der beiden Pulse, d.h. dem sich ggf. von Zeitintervall zu Zeitintervall ändernden Mittelwert der Spätverstellungen, entspricht und ein Maß für den jeweils erzielten Verdichtungsgrad ist.

Die Gleichspannung wird mit einem Spannunganzeiger 7a zur Anzeige gebracht.

Selbstverständlich erhält die Zündelektronik 4' über die augenblickliche Drehzahl hinaus noch weitere Informationen über für den Zündzeitpunkt wichtige Motorzustände, z.B. den Unterdruck im Saugrohr und die Motortemperatur.

Wenn, wie dies bei Verwendung des einfachen Spannungsanzeigers 7a der Fall ist, nur die aktuelle Phasenverschiebung bzw. Spätverstellung zur Anzeige gebracht wird, muß der Bedienungsmann zum Ende des Verdichtungsvorgangs hin diese Anzeige dauernd beobachten, um festzustellen, wann das erreichbare Maximum an Bodenverdichtung eingetreten ist, was er daran erkennt, daß sich die Anzeige, z.B. am Spannungsanzeiger 7a, nicht mehr ändert. Je nach Art des zu verdichtenden Bodens kann die Spätverstellung bzw. der Spätverstellwinkel im erreichbaren Endzustand der Bodenverdichtung aber durchaus verschieden sein, sodaß sich dieser Endzustand nicht zwangsläufig in einem von vornherein festliegenden Anzeigewert äußert. Häufig müssen große Flächen ein und derselben Bodenart verdichtet werden, und der Bedienungsmann muß dann über die ganze Fläche hinweg bzw. für jeden der Teilbereiche, die er jeweils in einem Stück bearbeitet und aus denen sich diese ganze Fläche dann zusammensetzt, den Zustand, daß sich die Spätverstellung nicht mehr ändert und damit das Maximum der Bodenverdichtung erreicht ist, durch andauerndes Beobachten der Veränderung der angezeigten aktuellen Spätverstellung feststellen, was über eine verhältnismäßig lange Zeit seine volle Aufmerksamkeit erfordert. Diese Feststellung kann dem Bedienungsmann dadurch wesentlich erleichtert werden, daß ihm die Möglichkeit geboten wird, zunächst nur für einen einzigen, relativ kleinen Teilbereich die vorgenannte Beobachtung durchzuführen und dann die für den Verdichtungsendzustand dieses Teilbereichs letzlich zur Anzeige kommende Größe der Spätverstellung abzuspeichern und weiterhin als Bezugsgröße anzuzeigen. Für den Rest der Verdichtungsarbeit über die gesamte Fläche hinweg braucht er dann nur noch die angezeigte aktuelle Spätverstellung mit dem Bezugswert zu vergleichen, und er kann die Verdichtungsarbeit jeweils beenden, wenn die aktuelle Anzeige mit dem Bezugswert übereinstimmt. Dies vereinfacht ihm die Beobachtung beträchtlich und erfordert bei weitem weniger Aufmerksamkeit.

In der Zeichnung sind über dem Spannungsanzeiger 7a zwei alternative Anzeigevorrichtungen 7b und 7c dargestellt, die jede für sich an dessen Stelle verwendbar sind und das vorstehend geschilderte Vorgehen ermöglichen.

Die Anzeigevorrichtung 7b stellt, wie der Spannungsanzeiger 7a jeweils in Form von Balken, oben den aus der anfänglichen abgeschlossenen Verdichtung des Probe-Teilbereichs der gesamten zu verdichtenden Fläche von gleicher Bodenart gewonnenen und abgespeicherten Bezugswert der Spätverstellung und darunter den bei der weiteren Verdichtungsarbeit jeweils aktuellen Grad der Spätverstellung dar. Im gezeigten Beispiel ist die Verdichtungsarbeit insgesamt oder für den gerade in Arbeit befindlichen Teilbereich beendet, wenn der letzte, am weitesten rechts befindliche schwarze Balken in der unteren Reihe unter dem schwarzen Balken in der oberen Reihe aufscheint.

Bei der Anzeigevorrichtung 7c handelt es sich um einen Vergleichsanzeiger, der wie ein Fenster-Diskriminator arbeitet und drei LEDs oder CCDs aufweist, die im Verlaufe der Verdichtungsarbeit der Reihe nach einzeln aufleuchten, wobei ↓, =, und ↑ bedeutet: geringere/gleiche/höhere Bodendichte als im Endzustand des Probebereichs.

Die Anzeige der Anzeigevorrichtungen 7b und 7c bezieht sich wie diejenige des Spannungsanzeigers 7a auf den Spät-Verstellwinkel.

Für Fälle, in denen dem Bedienungsmann die Bodenart hinsichtlich ihres Verhaltens und ihrer Eigenschaften beim Verdichten im Voraus bekannt ist, könnte auch die Möglichkeit zur Eingabe, Speicherung und gesonderten permanenten Anzeige eines von dem Bedienungsmann nach seinem Ermessen wählbaren Wertes der Anzeigegröße vorgesehen werden.

## Patentansprüche

1. Verfahren zum Feststellen und Anzeigen des Grades der von einem Bodenverdichtungsgerät während des Betriebes augenblicklich jeweils erreichten Bodendichte, **dadurch gekennzeichnet,** daß
a) das Verdichtungsgerät mittels eines Motors angetrieben wird, der von einem Überdrehzahl-Schutzregelkreis (4'), welcher bei Überschreiten einer vorbestimmten maximalen Drehzahl des Motors eine Spätverstellung der Zündimpulse bewirkt, gegen Überschreiten des vorbestimmten Drehzahlmaximalwertes gesichert ist, und
b) der Mittelwert der Spätverstellungen jeweils in einem Zeitintervall festgestellt und zur Bildung einer Anzeigegröße für den augenblicklich jeweils erreichten Bodenverdichtungsgrad herangezogen wird.

2. Verfahren zum Verdichten von größeren Bodenflächen gleicher Bodenart mit einem Bodenverdichtungsgerät unter Anwendung des Verfahrens nach Anspruch 1, **dadurch gekennzeichnet,** daß zunächst nur ein Probe-Teilbereich der Bodenfläche bis zum gewünschten Verdichtungsgrad verdichtet und der dabei erreichte Wert der Anzeigegröße als Bezugswert für die Verdichtung der restlichen Bodenfläche herangezogen wird, und daß die Verdichtungsarbeit beendet wird, wenn der aktuelle Wert der Anzeigegröße den Bezugswert erreicht hat.

3. Verfahren nach Anspruch 2, **dadurch gekennzeichnet,** daß der Bezugswert gespeichert und zusätzlich zu dem aktuellen Wert der Anzeigegröße permanent angezeigt wird.

4. Verfahren nach Anspruch 2 oder 3, **dadurch gekennzeichnet,** daß der Probe-Teilbereich bis zum maximal erreichbaren Verdichtungsgrad verdichtet wird.

## Claims

1. A process for detecting and indicating the degree of ground compactness momentarily reached by a ground compacting device during operation, characterised in that
a) the compacting device is driven by means of a motor, which is protected against exceeding the predetermined maximum rotational speed value by an overspeed protection control loop (4') which, when a predetermined maximum rotational motor speed is exceeded, causes a postponement of the ignition impulses, and
b) the average value of the postponements is respectively detected in a time interval and is referred to in order to form an indication variable for the respective momentarily reached degree of ground compactness.

2. A process for compacting larger areas of ground of the same soil type with a ground compacting device using the process according to claim 1, characterised in that only a small testing area of the ground surface is firstly compacted to the desired degree of compactness and the value of the indication variable thus achieved is referred to as a reference value for the compacting of the remaining ground surface, and that the compacting operation is ended when the actual value of the indication variable has reached the reference value.

3. A process according to claim 2, characterised in that the reference value is stored and is permanently displayed in addition to the actual value of the indication variable.

4. A process according to claim 2 or 3, characterised in that the small test region is compacted to the maximum achievable degree of compacting.

## Revendications

1. Procédé de détermination et d'indication du degré de la densité du sol obtenue instantanément au moyen d'un appareil de compression du sol, à chaque moment pendant le fonctionnement de l'appareil, caractérisé en ce que :
a) l'appareil de compression est entraîné au moyen d'un moteur qui est protégé au moyen d'un circuit de protection contre un dépassement de la vitesse contre un dépassement d'une vitesse de rotation maximale prédéterminée, ledit circuit de protection provoquant un retardement des impulsions d'allumage lors du dépassement de la vitesse maximale de rotation prédéterminée du moteur, et en ce que
b) la valeur moyenne des retardements est déterminée pendant un intervalle de temps et est mise à contribution pour la formation d'une grandeur indicative du degré de densité du sol atteint instantanément.

2. Procédé de compression de surfaces relativement grandes de toutes sortes de sols à l'aide d'un appareil de compression du sol en application du procédé selon la revendication 1, caractérisé en ce qu'on comprime d'abord seulement une zone partielle échantillon de la surface du sol jusqu'au degré de compression recherché, et que la valeur de la grandeur indicative alors obtenue en dernier lieu est utilisée comme valeur de référence pour la compression de la surface du sol restante, et en ce qu'on termine le travail de compression quand la valeur actuelle de la grandeur indicative a atteint la valeur de référence.

3. Procédé selon la revendication 2, caractérisé en ce que la valeur de référence est mise en mémoire et affichée en permanence, en supplément de la valeur actuelle de la grandeur indicative.

4. Procédé selon la revendication 2 ou 3, caractérisé en ce que la zone partielle échantillon est comprimée jusqu'au degré maximal de compression pouvant être atteint.
